# EUROPEAN PATENT SPECIFICATION

(11) **EP 1 813 584 B1**
(45) Date of publication and mention of the grant of the patent: **19.06.2013**
(21) Application number: 07380015.3
(22) Date of filing: 25.01.2007
(51) Int. Cl.: C05F 11/10, A01N 31/00

(54) **New metabolic and nutritional activator for plants**
Neuer Stoffwechsel- und Ernährungsaktivator für Pflanzen
Nouvel inducteur métabolique et nutritionnel pour plantes

(30) Priority: 26.01.2006 ES 200600178
(43) Date of publication of application: 01.08.2007
(73) Proprietor: Timac Agro España, S.A., 31160 Orcoyen, Navarra (ES)
(72) Inventor: Zamarreño Aregui, Angel, 31638 Eugui (Navarra) (ES); Garcia-Mina Freire, Jose M., 31170 Iza (Navarra) (ES); Houdusse, Fabrice, 31150 Mendigorria (Navarra) (ES); Casanova Portillo, Esther, 31015 Pamplona (Navarra) (ES)
(74) Representative: Del Santo Abril, Natividad

(56) References cited:
- EP-A- 0 965 269
- EP-A- 1 147 706
- EP-A- 1 442 664
- US-A- 3 671 212
- US-A1- 2005 014 698
- US-B1- 6 383 245
- US-B1- 6 461 664

## Description

The present invention relates to the use of a formulation with the ability to increase significantly the absorption and metabolization of mineral nutrients by plants. The main component of these formulations is 2-hydroxy-4-methyl thiobutanoic (HMTB) acid and its derivatives.

### Brief description of the figures

Figure 1 shows the effect of HMTB on the assimilation of nitrogen (N), magnesium (Mg), and iron (Fe) in a pepper plant. The result is expressed as percent over the control and is calculated from the contents of the elements in the aerial part and the dry matter of the aerial part.
Figure 2 shows the effect of HMTB on the assimilation of phosphorus (P), potassium (K), and calcium (Ca) in a pepper plant. The result is expressed as percentage over control, and is calculated from the contents of the elements in the aerial part and the dry matter of the aerial part.
Figure 3 shows the effect of HMTB on the production of dry matter (DM), the number of leaves, and the height of the pepper plants cultured in hydroponic conditions.

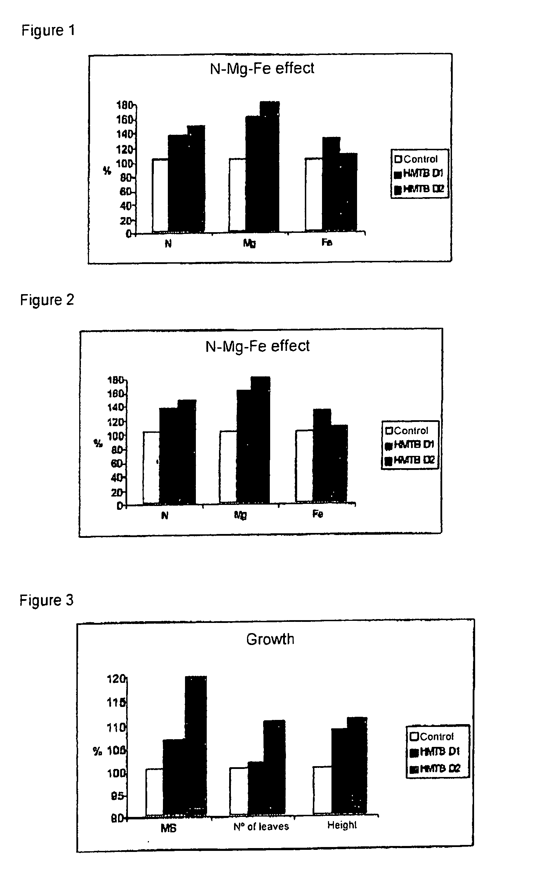

Figure 3 shows the result of the study on the effect of HMTB on the production of total proteins in a pepper plant.

### State of the art

One of the factors with the greatest influence in the development of cultures and consequently in the production/quality ratio is the appropriate assimilation of mineral nutrients by the plant and subsequent metabolization. No doubt a product which allows for a greater assimilation of the mineral elements and their better metabolization that is expressed in a greater development of the plant has an unquestionable interest.

Various patents or patent applications can be found in the state of the art (US Patent 4,579,962; US Patent 4,524,077; US2003144547; US5972300), that describe different methods for the preparation of HMTB and analogues of this acid. Its use is also protected in animals, mainly in ruminants, as an agent enhancing milk production (US6183786; US2005059739). However, there is no published information or filed patent on its use in plants as metabolic and nutritional activator.

US-6461664B describes a compound used in the agronomic and zootechnical field consisting of a chelate represented by the formula (CH3SCH2CH2CHOHCO) 2M. nH2O where M may be a bivalent metal and in particular Zn2+, Cu2+, Co2+, Mn2+, Ca2+ or Mg2+.

The methods currently employed to enhance plant development use mechanisms of hormonal nature, as described in the publication by Marschner, H., 1995. Mineral Nutrition of Higher Plants. Academic Press, London. 889 pp;

however, to date no formulations have been described to date which increase the assimilation of nutrients by plants and improve their metabolization, that use the properties of 2-hydroxy-4-methyl thiobutanoic acid (HMTB) (isomers D and L), its salts, esters, amides or ethers in position 2.

The inventors have discovered that, surprisingly, using 2-hydroxy-4-methyl- thiobutanoic acid (HMTB) (isomers D and L), its salts, esters, amides or ethers in position 2 in compositions such as those described in the present invention good results are obtained in the activation of absorption and metabolization of nutrients by plants without requiring the use of compositions stimulating hormonal mechanisms.

### Description of the invention

This invention describes the use of formulations comprising the compound 2-hydroxy-4-methyl thiobutanoic acid (HMTB) (isomers D and L), its salts, esters, amides or ethers in position 2, according to formula 1,

CH₃-S-CH₂-CH₂-CH(OR₁)-COR₂ I

where R₁ is selected from the group comprising hydrogen, alkyl rests (preferably methyl or ethyl) and aryl rests; while R₂ is selected from the group comprising hydroxy, amino, aryloxy and alkoxy moieties preferably methoxy or ethoxy moieties. When the rest is hydroxyl, acid salts are also considered with electropositive organic compounds, such as amines (for instance,ethanolamine).

It has been discovered that these products have the ability to increase significantly the assimilation of mineral nutrients, particularly nitrogen, magnesium and iron, and the metabolic processes associated with these elements, such as protein synthesis, chlorophyll synthesis and photosynthesis. As a result of this, it has been discovered that these compounds can increase plant development and culture quality. Formula I compounds can be included in the metabolic activator at any percentage, though the optimum contents ranges between 1 and 20%.

The metabolic activators described in this invention can contain, in addition to the compounds described in formula I, one or several compounds with auxin activity or precursors of auxin activity, such as indol acetic acid or tryptophan, or compounds with plant growth regulating activity, including cytokinins, ethylene, brassinosteroids, polyamines, salicylic acid, jasmonic acid, cyclic nucleotides, sucrose, nitric oxide and nitric oxide and abscisic acid precursors or givers. These components of either type can be included in the formulation at any percentage, though the optimum percentage ranges between 1 and 5% in weight.

In addition, the metabolic activator of this invention can contain one or several compounds with the ability to induce hormonal activity in plants such as indol, adenine, adenosine or isopentanol alcohol. The optimum content of these components in the new activator described herein ranges between 1 and 5% in weight, though this content can be increased.

In addition, the activator can include one or several compounds with plant growth stimulating activity such as humic substances, amino acids, seaweed extracts, lignosulfonates, compost plant residue extracts and vinasse or molasses of beetroot and cane. Although these compounds can be included in the formulation at any percentage, the optimum range is between 1 and 5% in weight.

Finally, one or several mineral nutrients can also be included as components of the activator. Therefore, nitrogen can be added, for instance by the addition of ammonia, ammonium nitrate, urea, ammonium sulfate or any salt containing nitrogen; phosphorus, for instance, using any salt of phosphoric, phosphorous, polyphosphoric or pyrophosphoric acid; potassium, for instance using potassium hydroxide, potassium sulfate, potassium chloride, potassium nitrate or potassium carbonate, or any salt containing potassium; magnesium, for instance using magnesium nitrate, magnesium sulfate or any salt containing magnesium; calcium, for instance using calcium nitrate, calcium chloride, and any salt containing calcium; sulfur, for instance using ammonium sulfate, magnesium sulfate or any salt or compound containing sulfur and trace elements, for instance using inorganic salts or organic compounds -chelates- of iron, copper, manganese, zinc, bore, molybdenum, titanium, nickel, silicon and cobalt. The optimum range of these mineral nutrients in the new activator described herein ranges between 5 and 10% in weight, though this content can be higher.

The formulations with activating properties referred to in this invention can be formulated in solid, dissolution or solid-adsorbed liquid state. In solid formulations, the solid is preferably of clay type, such as sepiolite, atapulgite, zeolite, or bentonite; though organic matters can be also used, such as peat; or organic polymers, such as polyacrylamide gels. In the case of liquid formulations, the preferential solvent is water. In both cases, the solid inert or the solvent are added to complete 100% in weight.

For the case of liquid formulations, the mixture of the components at the above described percentages is made in a reactor that can be made up of plastic or stainless steel, with helix stirring. The mixture can be obtained at any temperature, though it is preferably obtained in a range within 20-35 °C. The mixture can be also made at any pressure, though the preferred pressure is atmospheric pressure.

In solid formulations, the components are mixed in a blender (any blender could be used, for instance a paddle blender or Lodige). The mixture can be made at any temperature though it is preferably obtained within a range between 20 and 35°C. The mixture can also be performed at any pressure though the preferred pressure is atmospheric pressure. If any component is liquid, it would be applied during the mixing by crushing it over the solid components. The clays play the role of absorbing compound.

The metabolic activator object of this invention can be applied at any concentration range, with an optimum dose between 1 and 100 mg l⁻¹ (or kg⁻¹). The product can be applied foliarly over the aerial part of the plants or via the root either in solid formulations or in fertirrigation.

### Examples

To clarify the content of this invention, the following examples are provided that in no case should be considered as invention limiting. Examples 1 to 4 show various metabolic activators according to the present invention. All of them are obtained by directly mixing the components indicated in each case at room temperature and under continuous stirring. Example 5 shows the ability of the composition in example 1.
**Example 1 of formulation**
   10% of HMTB acid in liquid formulation
   90% water
**Example 2 of formulation**
   15% of HMTB acid in liquid formulation
   3% potassium hydroxide
   82% in water
**Example 3 of formulation**
   25% of methyl ester of HMTB acid
   75% sepiolite powder
**Example 4 of formulation**
   25% of HMTB acid in 88% liquid formulation
   5% hydrated iron sulfate (II)
   70% water
**Example 5 of formulation**
   20% HMTB of 88% liquid formulation
   1% indol
   5% fulvic acids
   74% water
**Example 6 of formulation**
   5% HMTB 88% liquid formulation
   0.5% indol
   10% urea
   84.5% sepiolite powder
**Example 7. Effect of the formulation of example 1 on nutrition and development of pepper plants cultivated in hydroponic conditions**
   The test performed consisted of the treatment of pepper plants -normally nourished using an appropriate nutrient solution-, with a solution containing HMTB according to the formulation described in example 1. The final dose of HMTB was 50 (D1) and 100 (D2) mg l⁻¹.

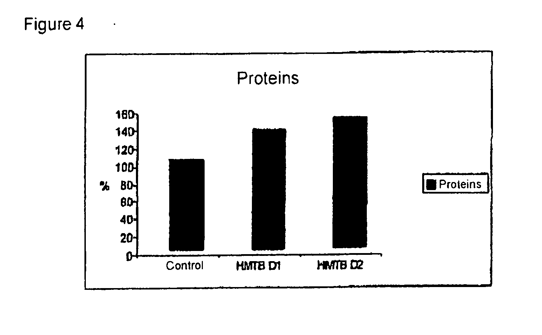

The plants were harvested at 4 weeks of starting the test.
The following results were found:
- Effect of nutritional activation
   Effect on the assimilation of magnesium-nitrogen-iron (Mg-N-Fe)
   As shown in Figure 1, highly significant increases were seen in the assimilation of these nutrients expressed for their highest content in the aerial part.
- Effect on the assimilation of phosphorus, calcium-potassium (P-Ca-K).
   Significant increases were also seen in the assimilation of P, Ca, and K as shown in Figure 2, though these were lower than in the case of Mg-N-Fe.
- Metabolic activation effect
   As a result of these nutritional increases, significant increases were seen in the development of plants, as shown in Figure 3. These effects on development were associated with significant improvements in some associated biochemical processes including the synthesis of total proteins as provided in Figure 4.

## Claims

1. Use of a formulation for improving the assimilation of mineral nutrients and activating the metabolism of these nutrients in the plants, wherein the formulation comprises one or several compounds represented by the general formula:
CH₃-S-CH₂-CH₂-CH(OR₁)-COR₂ I
wherein R₁ is selected from the group formed by hydrogen, alkyl rests, preferably methyl or ethyl, and aryl;
and wherein R₂ is selected from the group comprising hydroxy, amino, aryloxy and alkoxy moieties preferably methoxyl or ethoxy moieties, or an OX moiety where X is an electropositive organic compound, preferably ethanolamine

2. Use of a formulation according to claim 1 wherein the content of compound of formula I in said formulation is within the range of 1%-20% in weight.

3. Use of a formulation according to claim 1 wherein the formulation contains 2-hydroxy-4-methylbutanoic acid (CH₃-S-CH₂-CH₂-CH(OH)-COOH).

4. Use of a formulation according to claim 1 wherein the compound(s) defined according to general formula I is formulated with one or several compounds with auxin activity or precursors of auxin activity.

5. Use of a formulation according to claim 4 wherein the concentrations of the compounds with auxin activity or precursors of auxin activity are within the range of 1-5% in weight.

6. Use of a formulation according to claim 4 wherein the compounds with auxin activity or precursors of auxin activity are selected among indol-acetic acid and tryptophan.

7. Use of a formulation according to claim 1 wherein the compound(s) defined according to general formula I is formulated with one or several compounds with plant growth regulating activity.

8. Use of a formulation according to claim 7 wherein the compound with plant growth regulating activity is within the range 1-5% in weight.

9. Use of a formulation according to claim 7 wherein the compounds with plant growth regulatory activity are selected from the group formed by cytokinins, ethylene, brassinosteroids, polyamines, salicylic acid, jasmonic acid, cyclic nucleotides, sucrose, nitric oxide and precursors or givers of nitric and abscisic acid.

10. Use of a formulation according to claim 9 wherein the compound with plant growth regulating activity is nitric oxide or nitric oxide precursors.

11. Use of a formulation according to claim 1 wherein the compound(s) defined according to general formula I is formulated with one or several compounds with the ability to induce hormonal activity in plants.

12. Use of a formulation according to claim 11 wherein the concentration of the compounds inducing hormonal activity is within the range 1-5% in weight.

13. Use of a formulation according to claim 11 wherein the compounds inducing hormonal activity are selected from the group formed by indol, adenine, adenosine and isopentanol alcohol.

14. Use of a formulation according to claim 13 wherein the compound inducing hormonal activity is the indol.

15. Use of a formulation according to claim 1 wherein the compound(s) defined according to general formula I is formulated with one or several compounds with plant growth stimulating activity.

16. Use of a formulation according to claim 15 wherein the concentration of the compounds with plant growth stimulating activity is within the range 1-5%.

17. Use of a formulation according to claim 15 wherein the compounds with plant growth stimulating activity are selected from the group formed by humic substances, amino acids, seaweed extracts, lignosulfonates, compost plant residue extracts, and vinasse and molasses from beet and cane.

18. Use of a formulation according to claim 1 wherein the compound(s) defined according to general formula I also contain(s) one or several mineral nutrients.

19. Use of a formulation according to claim 18 wherein the concentration of the mineral nutrients is within the range 5-10%.

20. Use of a formulation according to claim 18 wherein the mineral nutrients are selected among ammonia, ammonium nitrate, urea, ammonium sulfate or any salt containing nitrogen; any salt of phosphoric, phosphorus, polyphosphoric or pyrophosphoric acid; potassium hydroxide, potassium sulfate, potassium chloride, potassium nitrate, potassium carbonate, or any salt containing potassium, magnesium nitrate, magnesium sulfate or any salt containing magnesium; calcium nitrate, calcium chioride or any salt containing calcium; ammonium sulfate, magnesium sulfate or any salt or compound containing sulfur; and inorganic salts or iron, copper, manganese, zinc, bore, molybdenum, titanium, nickel, silicon and cobalt chelates.

21. Use of a formulation according to any of claims 1-20 wherein said formulation can be formulated in liquid, solid state or liquid absorbed in absorbent materials.

22. Use of a formulation according to claim 21 wherein the absorbing materials are selected among clays, organic matters or organic polymers.

23. Use of a formulation according to claim 21 wherein said formulation is applied in a dose range between 1 and 100 mg I⁻¹ (or kg⁻¹).

## Patentansprüche

1. Verwendung einer Rezeptur zur Verbesserung der Aufspaltung mineralischer Nährstoffe und zur Anregung des Stoffwechsels bezüglich dieser Nährstoffe in den Pflanzen, worin die Rezeptur einen oder mehrere Stoffe umfasst, welche durch die allgemeine Formel repräsentiert wird:
CH₃-S-CH₂-CH₂-CH(OR₁)-COR₂ I
worin R₁ aus der aus Wasserstoff, Alkylresten, vorzugsweise Methyl oder Ethyl und Aryl bestehenden Gruppe ausgesucht wurde;
und worin R₂ aus der aus Hydroxy-, Amino-, Aryloxy und Alkoxyresten bestehenden Gruppe ausgesucht wurde, vorzugsweise Methoxyl- oder Ethoxyresten oder einem OX-Rest, wo X ein elektropositiver organischer Stoff ist, vorzugsweise Ethanolamin.

2. Verwendung einer Rezeptur, entsprechend Anspruch 1, worin der Anteil des Stoffes des Rezepts I in der besagten Rezeptur im Bereich von 1%-20% des Gewichts liegt.

3. Verwendung einer Rezeptur, entsprechend Anspruch 1, worin die Rezeptur 2-Hydroxy-4-Methylbuttersäure
(CH₃-S-CH₂-CH₂-CH(OH)-COOH) enthält.

4. Verwendung einer Rezeptur, entsprechend Anspruch 1, worin der (die) Stoff(e) entsprechend der allgemeinen Formel I mit einem oder mehreren Stoffen mit Auxinaktivität oder biologischen Vorstufen von Auxinaktivität gestaltet ist (sind).

5. Verwendung einer Rezeptur, entsprechend Anspruch 4, worin die Konzentrationen der Stoffe mit Auxinaktivität oder biologischen Vorstufen von Auxinaktivität im Bereich von 1-5% des Gewichts liegen.

6. Verwendung einer Rezeptur, entsprechend Anspruch 4, worin die Stoffe mit Auxinaktivität oder biologischen Vorstufen von Auxinaktivität aus Indol-Essigsäure und Tryptophan ausgewählt sind.

7. Verwendung einer Rezeptur, entsprechend Anspruch 1, worin der (die) Stoff(e) entsprechend der allgemeinen Formel I mit einem oder mehreren Stoffen mit wachstumsfördernder Aktivität in Pflanzen gestaltet ist (sind).

8. Verwendung einer Rezeptur, entsprechend Anspruch 7, worin der Stoff mit wachstumsfördernder Aktivität in Pflanzen im Bereich von 1-5% des Gewichts liegt.

9. Verwendung einer Rezeptur, entsprechend Anspruch 7, worin die Stoffe mit wachstumsfördernder Aktivität in Pflanzen aus der Gruppe ausgesucht wurde, die von Cytokininen, Ethylenen, Brassinosteroiden, Polyaminen, Salicylsäure, Jasmonsäure, zyklischen Nukleotiden, Saccharose, Stickoxid und den Ausgangsstoffen oder Abgebende von Salpeter- und Abscisinsäure gebildet wird.

10. Verwendung einer Rezeptur, entsprechend Anspruch 9, worin der Stoff mit wachstumsfördernder Aktivität in Pflanzen Stickoxid oder ein Ausgangsstoff von Stickoxid ist.

11. Verwendung einer Rezeptur, entsprechend Anspruch 1, worin der (die) Stoff(e) entsprechend der allgemeinen Formel I mit einem oder mehreren Stoffen mit der Fähigkeit gestaltet ist (sind), hormonelle Aktivität in Pflanzen herbeizuführen.

12. Verwendung einer Rezeptur, entsprechend Anspruch 11, worin die Konzentration von Stoffen, welche hormonelle Aktivität herbeiführen, im Bereich von 1-5% des Gewichts liegen.

13. Verwendung einer Rezeptur, entsprechend Anspruch 11, worin die Stoffe, welche hormonelle Aktivität herbeiführen, aus der Gruppe ausgesucht wurde, die von Indol, Adenin, Adenosin und Isopentanolalkohol gebildet wird.

14. Verwendung einer Rezeptur, entsprechend Anspruch 13, worin der Stoff, welcher hormonelle Aktivität herbeiführt, das Indol ist.

15. Verwendung einer Rezeptur, entsprechend Anspruch 1, worin der (die) Stoff(e) entsprechend der allgemeinen Formel I mit einem oder mehreren Stoffen mit wachstumsfördernder Aktivität in Pflanzen gestaltet ist (sind).

16. Verwendung einer Rezeptur, entsprechend Anspruch 15, worin die Konzentration der Stoffe, welche wachstumsfördemde Aktivität in Pflanzen herbeiführen, im Bereich von 1-5% liegt.

17. Verwendung einer Rezeptur, entsprechend Anspruch 15, worin die Stoffe, die mit der Fähigkeit gestaltet sind, wachstumsfördernde Aktivität in Pflanzen herbeizuführen, aus der Gruppe ausgesucht wurden, die von Huminstoffen, Aminosäuren, Algenextrakten, Lignosulfonaten, Zugaben aus Restmengen von Kompostierungsanlagen sowie Vinasse und Melasse aus Zuckerrüben und Rohrzucker gebildet wird.

18. Verwendung einer Rezeptur, entsprechend Anspruch 1, worin der (die) Stoff(e) entsprechend der allgemeinen Formel I auch ein oder mehrere mineralische Nährstoffe enthält (enthalten).

19. Verwendung einer Rezeptur, entsprechend Anspruch 18, worin die Konzentration der mineralischen Nährstoffe im Bereich 5-10% liegt.

20. Verwendung einer Rezeptur, entsprechend Anspruch 18, worin die mineralischen Nährstoffe aus den Stoffen Ammoniak, Ammoniumnitrat, Harnstoff, Ammoniumsulfat oder irgendeinem Stickstoff enthaltenden Salz; irgendeinem Salz der Phosphorsäure, von Phosphor, von Polyphosphor oder Pyrophosphorsäure; aus Kaliumhydroxid, Kaliumsulfat, Kaliumchlorid, Kaliumnitrat, Kaliumkarbonat oder irgendeinem Salz, das Kalium, Magnesiumnitrat, Magnesiumsulfat oder irgendeinem Magnesium enthält; aus Calciumnitrat, Calciumchlorid oder irgendeinem Calcium enthaltenden Salz; aus Ammoniumsulfat, Magnesiumsulfat oder irgendeinem Salz oder anderen Stoff, der Schwefel enthält; und aus anorganischen Salzen oder Eisen, Kupfer, Mangan, Zink, Bor, Molybdän, Titan, Nickel, Silicon und Cobaltchelaten ausgewählt werden.

21. Verwendung einer Rezeptur, entsprechend den Ansprüchen 1-20, worin die besagte Rezeptur in flüssigem und festem Zustand oder als Flüssigkeit in absorptionsfähigen Materialien absorbiert, ausgearbeitet werden kann.

22. Verwendung einer Rezeptur, entsprechend Anspruch 21, worin die absorbierenden Materialien unter Tonerden, organischen Substanzen oder Organokunststoffen ausgesucht werden.

23. Verwendung einer Rezeptur, entsprechend Anspruch 21, worin die besagte Rezeptur in einem Dosisbereich zwischen 1 und 100 mg I⁻¹ (oder kg⁻¹) angewendet wird.

## Revendications

1. Utilisation d'une formulation pour améliorer l'assimilation de nutriments minéraux et pour activer le métabolisme de ces nutriments chez les plantes, dans laquelle la formulation comprend un ou plusieurs composants représentés par la formule général :
CH₃-S-CH2-CH₂-CH(OR₁)-COR2 I
dans laquelle R₁ est sélectionnée du groupe formé par l'hydrogène, des motifs alkyles, préférablement du méthyle ou de l'éthyle, et l'aryle ;
et dans laquelle R₂ est sélectionné parmi le groupe comprenant des motifs hydroxy, amino, aryloxy et alcoxy préférablement des motifs méthoxy ou éthoxy, ou un motif OX où X est un composant organique électropositif, préférablement de l'éthanolamine

2. Utilisation d'une formulation selon la revendication 1 dans laquelle la teneur de composants de la formule I dans ladite formulation est comprise dans l'intervalle 1%-20% en poids.

3. Utilisation d'une formulation selon la revendication 1 dans laquelle la formulation contient de l'acide 2-hydroxy-4-méthylbutanoïque (CH₃-S-CH₂-CH₂-CH(OH)-COOH).

4. Utilisation d'une formulation selon la revendication 1 dans laquelle le composant ou les composants définis selon la formule générale I sont formulés avec un ou plusieurs composants ayant une activité auxinique ou des précurseurs d'activité auxinique.

5. Utilisation d'une formulation selon la revendication 4 dans laquelle les concentrations des composants ayant une activité auxinique ou des précurseurs d'activité auxinique sont comprises dans l'intervalle 1-5% en poids.

6. Utilisation d'une formulation selon la revendication 4 dans laquelle les composants ayant une activité auxinique ou les précurseurs d'activité auxinique sont sélectionnés parmi l'acide indole-acétique et le tryptophane.

7. Utilisation d'une formulation selon la revendication 1 dans laquelle le composant ou les composants définis selon la formule générale I sont formulés avec un ou plusieurs composants ayant une activité régulatrice pour la croissance des plantes.

8. Utilisation d'une formulation selon la revendication 7 dans laquelle le composant ayant une activité régulatrice pour la croissance des plantes est compris dans l'intervalle 1-5% en poids.

9. Utilisation d'une formulation selon la revendication 7 dans laquelle les composants ayant une activité régulatrice pour la croissance des plantes sont sélectionnés du groupe formé par les cytokinines, l'éthylène, les brassinostéroïdes, les polyamines, l'acide salicylique, l'acide jasmonique, les nucléotides cycliques, le sucrose, l'oxyde nitrique et les précurseurs ou donneurs de l'acide nitrique et abscissique.

10. Utilisation d'une formulation selon la revendication 9 dans laquelle le composant ayant une activité régulatrice pour la croissance des plantes est de l'oxyde nitrique ou des précurseurs de l'oxyde nitrique.

11. Utilisation d'une formulation selon la revendication 1 dans laquelle le composant ou les composants définis selon la formule générale I sont formulés avec un ou plusieurs composants ayant l'habilité d'induire l'activité hormonale des plantes.

12. Utilisation d'une formulation selon la revendication 11 dans laquelle la concentration des composants induisant l'activité hormonale est comprise dans l'intervalle 1-5% en poids.

13. Utilisation d'une formulation selon la revendication 11 dans laquelle les composants induisant l'activité hormonale sont sélectionnés du groupe formé par l'indole, l'adénine, l'adénosine et l'alcool isopentanol.

14. Utilisation d'une formulation selon la revendication 13 dans laquelle le composant induisant l'activité hormonale est l'indole.

15. Utilisation d'une formulation selon la revendication 1 dans laquelle le composant ou les composants définis selon la formule générale I sont formulés avec un ou plusieurs composants ayant une activité régulatrice pour la croissance des plantes.

16. Utilisation d'une formulation selon la revendication 15 dans laquelle la concentration des composants ayant une activité régulatrice pour la croissance des plantes est comprise dans l'intervalle 1-5%.

17. Utilisation d'une formulation selon la revendication 15 dans laquelle les composants ayant une activité régulatrice pour la croissance des plantes sont sélectionnés du groupe formé par des substances humiques, des aminoacides, des extraits d'algues marines, des lignosulfonates, des extraits de résidus végétaux compostés, et des vinasses et mélasses de betterave et de canne.

18. Utilisation d'une formulation selon la revendication 1 dans laquelle le composant ou les composants définis selon la formule générale I contiennent aussi un ou plusieurs nutriments minéraux.

19. Utilisation d'une formulation selon la revendication 18 dans laquelle la concentration des nutriments minéraux est comprise dans l'intervalle 5-10%.

20. Utilisation d'une formulation selon la revendication 18 dans laquelle les nutriments minéraux sont sélectionnés parmi l'ammoniac, le nitrate d'ammonium, l'urée, le sulfate d'ammonium ou n'importe quel sel contenant de l'azote ; n'importe quel sel de l'acide phosphorique, phosphoreux, polyphosphorique ou pyrophosphorique ; de l'hydroxyde de potassium, du sulfate de potassium, du chlorure de potassium, du nitrate de potassium, le carbonate de potassium, ou n'importe quel sel contenant du potassium, du nitrate de magnésium, du sulfate de magnésium ou n'importe quel sel contenant du magnésium ; du nitrate de calcium, du chlorure de calcium ou n'importe que sel contenant du calcium ; du sulfate d'ammonium, du sulfate de magnésium ou n'importe quel sel ou composant contenant du soufre ; et des sels inorganiques ou des chélates de fer, cuivre, manganèse, zinc, bore, molybdène, titane, nickel, silicium et du cobalt.

21. Utilisation d'une formulation selon quelconque des revendications 1-20 dans laquelle ladite formulation peut être formulée à l'état liquide ou solide ou bien liquide absorbée en des matériaux absorbants.

22. Utilisation d'une formulation selon la revendication 21 dans laquelle les matériaux absorbants sont sélectionnés parmi les argiles, les matériaux organiques ou les polymères organiques.

23. Utilisation d'une formulation selon la revendication 21 dans laquelle ladite formulation est appliquée dans un intervalle de dosage comprise entre 1 et 100 mg I⁻¹ (ou kg⁻¹).
